# EUROPÄISCHE PATENTSCHRIFT

(11) **EP 1 082 879 B1**
(45) Veröffentlichungstag und Bekanntmachung des Hinweises auf die Patenterteilung: **22.02.2006**
(21) Anmeldenummer: 99926416.1
(22) Anmeldetag: 26.05.1999
(51) Int. Cl.: H05B 3/36

(54) **HEIZMATTE**
ELECTRIC BLANKET
COUVERTURE CHAUFFANTE

(30) Priorität: 26.05.1998 DE 19823496
(43) Veröffentlichungstag der Anmeldung: 14.03.2001
(73) Patentinhaber: Latec AG, 8702 Zollikon (CH)
(72) Erfinder: OPPITZ, Hans, A-6068 Mils (AT)
(74) Vertreter: Kador & Partner
(86) Internationale Anmeldenummer: PCT/EP1999/003615
(87) Internationale Veröffentlichungsnummer: WO 1999/062302

(56) Entgegenhaltungen:
- WO-A-96/36057
- WO-A-98/09478
- US-A- 3 627 981
- US-A- 4 700 054
- US-A- 4 888 472
- US-A- 5 004 895

## Beschreibung

Die vorliegende Erfindung betrifft eine Heizmatte, insbesondere eine Heizmatte für die Verwendung als Bettheizung.

Heizmatten finden vielfach Anwendung als Bettheizungen. Hierbei werden diese auf die Matratze gelegt oder in diese eingearbeitet, um z.B. in Krankenhäusern Patienten auf der Intensivstation eine ausreichende Menge an Wärme zuzuführen. Abhängig von der Verletzung der Patienten kann aber eine Erwärmung verschiedener Bereiche des Körpers unerwünscht sein. Überdies muß bei Heizmatten, die in unmittelbaren Kontakt mit dem menschlichen Körper kommen, sichergestellt sein, daß ein Durchbrennen des Heizelementes, lokale Temperaturerhöhungen und Stromschläge vermieden werden.

Aus US 3,627,981 sind Flächenheizelemente bekannt, die flexibel ausgearbeitet sind und ein Widerstandselement für die Umwandlung elektrischer Energie in thermische Energie enthalten. Vorzugsweise wird dabei ein Polyestervlies als Widerstandsheizelement verwendet. Metallbänder, die mit dem Vlies in flachen Oberflächenkontakt stehen, dienen dabei als Stromversorgungsleitungen.

Aus US 5,004,895 sind Heizvorrichtungen für Fußböden in Innenräumen bekannt, die eine leitfähige Kunststoffschicht aufweisen, welche zwischen zwei Isolierschichten eingebettet ist. Die Heizvorrichtung ist dabei für den Betrieb bei geringer elektrischer Stromstärke geeignet.

Aus US 4,700,054 ist die Verwendung eines Materials mit positiven Temperaturkoeffizienten (PTC) in einem elektrischen Heizapparat bekannt. Der elektrische Heizapparat umfasst dabei vorzugsweise ein PTC-Element, beispielsweise ein leitendes Polymer, um die Heizung selbstregulierend zu gestalten. Das PTC-Element selber kann in Form von Fasern innerhalb eines Gewebes oder als Schicht, welche eine der Elektroden umgibt, oder als flächenhaftes Element in welches das Gewebe eingebettet ist, ausgearbeitet sein.

Aufgabe der vorliegenden Erfindung ist es, eine Heizmatte zu schaffen, die eine gezielte Erwärmung von Teilen der Matte erlaubt, und die ohne Sicherheitsrisiko in unmittelbarem Kontakt mit dem menschlichen Körper verwendet werden kann.

Der Erfindung liegt die Erkenntnis zugrunde, daß diese Aufgabe durch eine Heizmatte gelöst werden kann, bei der ein Widerstandsheizelement mit einer geeigneten Widerstandsmasse und über der Fläche der Heizmatte verteilten Stromversorgungsleitungen verwendet wird.

Die Aufgabe wird erfindungsgemäß durch eine Heizmatte mit einem flächigen Widerstandsheizelement, dessen Widerstandsmasse ein elektrisch leitendes Polymer mit einem positiven Temperaturkoeffizienten des elektrischen Widerstandes umfaßt, gelöst, wobei in dem Widerstandsheizelement drei oder mehr Versorgungsleitungen vorgesehen sind, die wahlweise mit Strom beaufschlagt werden können, und die Widerstandsmasse, ein Gitter umfasst, wobei die Fäden des Gitters aus einem Kunststoff aus dem elektrisch leitenden Polymer gebildet sind oder die Fäden aus einem anderen Material bestehen und mit diesem Kunststoff beschichtet sind.

Im Folgenden werden die Stromversorgungsleitungen auch als Elektroden bezeichnet und die durch die Widerstandsmasse gebildete Fläche auch als Widerstandsschicht bezeichnet.

Bei der erfindungsgemäßen Heizmatte wird die Wärme durch den an den Stromversorgungsleitungen angelegten Strom erzeugt. Dieser durchfließt die Widerstandsmasse und erwärmt diese. In der erfindungsgemäßen Heizmatte sind mindestens drei Stromversorgungsleitungen vorgesehen. Werden zwei der Stromversorgungsleitungen an die Stromquelle angeschlossen, so fließt durch den Bereich der Widerstandsmasse, der zwischen diesen beiden Stromversorgungsleitungen liegt, ein Heizstrom, der diesen Bereich erwärmt. Bei drei Stromversorgungsleitungen wird somit die Möglichkeit gegeben, durch das jeweilige Anschließen von zwei Stromversorgungsleitungen an die Stromquelle die Größe und/oder die Lage des Bereiches der Heizmatte auszuwählen, die erwärmt werden soll. Auch die zu erzielende Temperatur kann durch die Größe des stromdurchflossenen Bereiches beeinflußt und präzise eingestellt werden. Mit der Heizmatte ist somit eine Zonenerwärmung auf unterschiedliche Temperaturen möglich. Liegt bei dieser Ausführungsform eine nicht mit Strom beaufschlagte Elektrode zwischen einem mit Strom beaufschlagten Elektrodenpaar, so dient die nicht kontaktierte Elektrode als Leiter und sorgt für eine gleichmäßige Verteilung des Stroms über die Breite der Heizmatte.

Bei einer Widerstandsmasse, die ein elektrisch leitendes Polymer umfaßt, ist zudem eine hohe Flexibilität gewährleistet. Die Widerstandsmasse kann somit mechanischen Belastungen standhalten, ohne daß sich deren elektrischen Eigenschaften ändern. Insbesondere kann es bei der erfindungsgemäß verwendeten Widerstandsmasse nicht zu Abrissen kommen, wie sie bei Heizelementen, die mit z.B. Rußgittern in der Widerstandsmasse arbeiten, auftreten. Lokale Temperaturerhöhungen, die bei solchen Abrissen auftreten, sind in der erfindungsgemäßen Heizmatte nicht zu befürchten. Die Heizmatte kann daher ohne Bedenken in unmittelbarem Kontakt mit dem menschlichen Körper verwendet werden.

Die Widerstandsmasse läßt sich zudem leicht verarbeiten, so daß die Stromversorgungsleitungen leicht in die Widerstandsmasse eingebracht oder an dieser angebracht werden können.

Ein besonderer Vorteil der erfindungsgemäßen Heizmatte ist, daß der durch den Heizstrom erwärmte Bereich Wärme über die gesamte Oberfläche abgeben kann. Dieser Bereich kann unmittelbar mit dem menschlichen Körper in Kontakt treten, wodurch diesem in dem definierten Bereich gleichmäßig Wärme zugeführt wird.

Ein Heizelement mit einer Widerstandsmasse, die ein elektrisch leitendes Polymer umfaßt, kann mit geringen Spannungen von z.B. 12 oder 24 V betrieben werden da die eingebrachte Heizenergie ideal genutzt wird. Ein menschlicher Körper kann somit gefahrlos mit dem Widerstandsheizelement in Kontakt gebracht werden. Die geringe Spannung, die zur Erwärmung des Widerstandsheizelementes benötigt wird, hat weiterhin den Vorteil, daß eine Isolierschicht auf der dem Körper zugewandten Seite nicht erforderlich ist. Das Widerstandsheizelement in der Heizmatte kann vielmehr unmittelbar oder vorzugsweise durch eine Textilschicht mit dem Körper in Kontakt stehen. Durch diese Nähe des Körpers zu dem Widerstandsheizelement kann eine Tiefenwirkung, die durch Infrarotstrahlung ausgelöst wird, ideal genutzt werden. Die Entstehung der IR-Strahlung wird im folgenden beschrieben.

Bei Verwendung des elektrisch leitenden Polymers dient das Widerstandsheizelement als "schwarzer Körper", der Strahlungen aller Wellenlänge abgeben kann. Mit abnehmender Temperatur verschiebt sich die Wellenlänge der abgegebenen Strahlung immer mehr zum unsichtbaren Infrarot. Bei relativ geringer Temperatur des Heizelementes in der Heizmatte kann somit eine Erwärmung eines auf oder unter der Heizmatte liegenden Körpers durch die Infrarotstrahlung, vorzugsweise einheitlicher Wellenlänge, erzielt werden, die in den Körper eindringt.

Gemäß einer Ausführungsform weist das elektrisch leitende Polymer einen positiven Temperaturkoeffizienten des elektrischen Widerstandes auf. Aufgrund des positiven Temperaturkoeffizienten des elektrischen Widerstandes des in der Widerstandsmasse verwendeten Polymers wird eine vollautomatische Begrenzung der Temperatur erreicht.

Gemäß einer bevorzugten Ausführungsform der erfindungsgemäßen Heizmatte erstreckt sich die Widerstandsmasse des Widerstandsheizelementes in Längsrichtung der Heizmatte und die Stromversorgungsleitungen erstrecken sich in Breitenrichtung durch die Widerstandsmasse, so daß der an den Stromversorgungsleitungen angelegte. Strom die Widerstandsmasse senkrecht zu der Dicke der Widerstandsmasse durchfließt.

Bei Vorsehen einer solchen Anordnung der Stromversorgungsleitungen in einem flächigen Widerstandsheizelement durchfließt der Strom die Widerstandsmasse von einer Stromversorgungsleitung zur nächsten kontaktierten Stromversorgungsleitung. Der so vom Strom durchflossene Bereich der Widerstandsmasse wird erwärmt und gibt an die Umgebung Wärme ab. Der erwärmte Bereich wird bei dieser Ausführungsform durch die kontaktierten Stromversorgungsleitungen definiert und begrenzt. Insbesondere bei Verwendung der Heizmatte als Bettheizung können z.B. lediglich der Kopf-oder Fußbereich der Heizmatte durch Anlegen des Strom an die jeweilig vorgesehenen Stromversorgungsleitungen erwärmt werden.

Wenn die Widerstandsmasse ein Gitter umfasst hat das den Vorteil, daß die Heizmatte eine hohe Flexibilität aufweist. Bei einer Belastung der Heizmatte kommt es aufgrund der definierten Kreuzungspunkte des Gitters nicht zur Relativveränderung des Abstandes zwischen den einzelnen Fäden der Widerstandsmasse. Eine lokale Temperaturerhöhung kann somit vermieden werden. Zusätzlich ist durch die Wahl eines Gitters als Widerstandsmasse für das Heizelement eine gleichmäßige Verteilung des angelegten Heizstromes möglich, da dieser die gesamte Widerstandsmasse durchfließen kann. Tritt aufgrund äußerer Umstände, z.B. durch Sitzen oder Liegen auf der Heizmatte, eine lokale Temperaturerhöhung auf, so wird die Leistung bei einem positiven Temperaturkoeffizienten des Widerstandes in diesem Bereich verringert. Die angelegte Spannung wird dann über die abzweigenden bzw. benachbarten Fäden des Gitters, deren Widerstand geringer ist, zu der nächsten kontaktierten Stromversorgungsleitung weitergeleitet.

Umfaßt die Widerstandsmasse der Heizmatte ein Gitter, bei dem die Fäden mit einem Kunststoff aus dem elektrisch leitenden Polymer mit einem positiven Temperaturkoeffizienten des elektrischen Widerstandes beschichtet sind, so können Grundmaterialien wie Polyamid oder Polyester für die Fäden verwendet werden.

Durch Verwendung eines Gitters als Widerstandsmasse sind zudem Öffnungen in dem Heizelement vorhanden. Diese sind insbesondere bei Verwendung der Heizmatte als Bettheizung vorteilhaft, da dadurch eine gute Dampfdiffusion sichergestellt werden kann. Schweiß z.B. kann durch die Heizmatte hindurchtreten und so vom Körper weggeführt werden. Zudem kann durch geeignete Wahl der Fäden eine gleichmäßige Strom- und damit Temperaturverteilung erzielt werden.

Schließlich können in eine Widerstandsmasse, die als Gitter ausgebildet ist, Stromversorgungsleitungen leicht eingeführt werden und weisen einen guten Kontakt zur Widerstandsmasse auf.

Gemäß einer weiteren Ausführungsform kann die Widerstandsmasse einen nichtlinearen positiven Temperaturkoeffizienten des elektrischen Widerstandes mit einem Kennlinienknick aufweisen. Durch diese spezielle Eigenschaft kann eine vorbestimmte Maximaltemperatur der Heizmatte erzielt werden. Diese Maximaltemperatur wird dadurch eingestellt, daß es bei Überschreiten der Temperatur zu einer wesentlichen Erhöhung des elektrischen Widerstandes der Widerstandsmasse kommt, wodurch automatisch die zugeführte Leistung reduziert und eine weitere Erwärmung unterbunden wird.

Die Stromversorgungsleitungen der erfindungsgemäßen Heizmatte können durch Lahnbänder gebildet werden. Die Verwendung solcher Bänder als Stromversorgungsleitungen bringt den Vorteil, daß auch bei Vorsehen mehrerer Stromversorgungsleitungen in der Heizmatte deren Flexibilität nicht beeinträchtig wird. Lahnbänder können zudem leicht in die Widerstandsmasse eingebracht werden und z.B. bei einer gitterförmigen Ausführung der Widerstandsmasse sogar in dieser verarbeitet sein.

Die Stromversorgungsleitungen sind vorzugsweise parallel zueinander in einem Abstand von mindestens 4 cm, vorzugsweise 5 - 10 cm, angeordnet. Bei dieser Ausführungsform können die zu beheizenden Bereiche klein gewählt werden. Sofern die Stromversorgungsleitungen parallel zueinander angeordnet sind, wird ein Kurzschluß durch Kontakt zwischen diesen zuverlässig vermieden.

Die erfindungsgemäße Heizmatte kann so ausgestattet sein, daß Einrichtungen vorgesehen sind, um die Stromversorgungsleitungen an eine einzige Stromquelle anschließen zu können. Diese Einrichtung kann in Form eines Verteilers ausgestattet sein, mit dem einzelne Stromversorgungsleitungen mit Strom beaufschlagt werden können und andere Stromversorgungsleitungen nicht kontaktiert werden. Durch eine solche Einrichtung kann die Wahl der zu erwärmenden Bereiche und der einzustellenden Temperatur, die durch die Heizleistung erzeugt wird, leicht geändert werden und auch im Einsatz z.B. bei Verwendung als Bettheizung den Bedürfnissen des Patienten angepaßt werden. Als Verteiler können elektrische Schalter verwendet werden. Es liegt aber auch im Rahmen der Erfindung, manuelle Kontaktierungsmöglichkeiten, z.B. Druckknöpfe, an jeder Elektrode vorzusehen. Dies bietet sich bei Bettheizungen an, bei denen im Normalfall die eingestellten Temperaturen nicht verändert werden müssen.

Die Stromversorgungsleitungen werden mit Kleinspannung bis 48 V, vorzugsweise mit Gleichstrom aus Akkumulatoren und/oder Netzgeräten mit 12 oder 24 V beaufschlagt. Dadurch wird jeglicher Elektrosmog vermieden und die in Kliniken eingesetzten Überwachungsgeräte wie z.B. EEG, EKG u.a. nicht gestört.

Vorzugsweise werden die Stromversorgungsleitungen mit Gleichstrom beaufschlagt. Dadurch dient die erfindungsgemäße Heizmatte zugleich als abschirmung von elektrischen Wechselfeldern. Zudem kann die Anspeisung der Stromversorgungen geerdet werden.

Die Heizmatte kann erfindungsgemäß an nur einer Oberfläche des Widerstandsheizelementes eine Isolierschicht oder eine Reflexionsschicht aufweisen. Üblicherweise wird die Temperaturdifferenz zwischen dem durch die Heizmatte zu beheizenden, insbesondere menschlichen, Körper und der Heizmatte geringer sein als die Temperaturdifferenz der Heizmatte zur Umgebung oder zu einer Unterlage. Aufgrund dieser unterschiedlichen Temperaturdifferenz wird die Wärme vorzugsweise an die Umgebung bzw. Unterlage abgegeben werden. Um diese Wärmeabgabe, die einen Wärmeverlust darstellt, zu vermeiden, kann an der dem Körper abgewandten Seite die Isolationsschicht vorgesehen sein. Da bei der erfindungsgemäßen Heizmatte nur auf einer Seite eine Isolierschicht vorgesehen ist und die Wärme somit nur in eine Richtung abgegeben wird, wird gegenüber einem Heizelement, das an beiden Seiten eine Isolierschicht aufweist, nur die halbe Heizleistung benötigt, um die gleichen Temperaturen an den Körper abgeben zu können. Das Heizelement kann somit mit geringeren Spannungen betrieben werden. Es können Spannungen bis zu 48 V an die Heizmatte angelegt werden. Vorzugsweise wird die Heizmatte aber mit Spannungen von 12 bzw. 24 V betrieben. Aufgrund dieser geringen Spannungen kann die Heizmatte näher an den Körper und vorzugsweise nur durch eine textile Abdeckung von ihm getrennt sein. Durch eine Reflexionsschicht kann darüberhinaus die Wärmestrahlung noch in Richtung des Körpers gelenkt, und somit die Wärmenutzung der Heizmatte verbessert werden.

Die vorliegende Erfindung wird anhand der beiliegenden Figuren erläutert.

Es zeigen:
- Figur 1:: Die Draufsicht auf eine erfindungsgemäße Heizmatte;
- Figur 2:: Teilschnitt durch ein erfindungsgemäß verwendetes Widerstandsheizelement:
- Figur 3:: Schnitt durch eine weitere Ausführungsform der erfindungsgemäßen Heizmatte;
- Figur 4:: Abhängigkeit der Temperatur und Leistungsaufnahme von der Zeit bei erfindungsgemäßen Heizmatten.

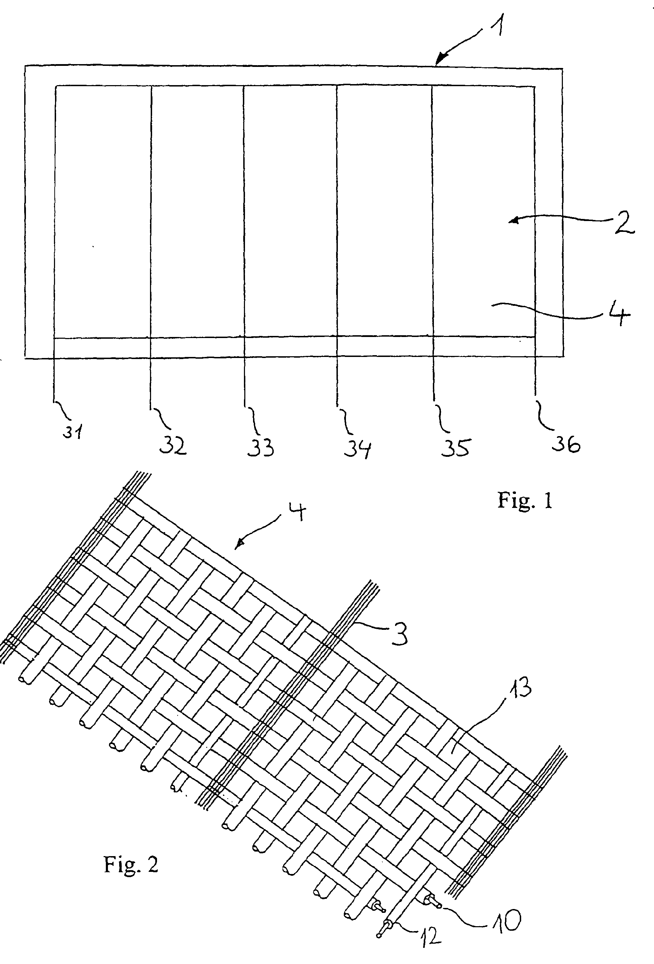

In Figur 1 weist das Widerstandsheizelement 2 der Heizmatte 1 sechs Stromversorgungsleitungen 31-36 auf. Die Stromversorgungsleitungen 31-36 sind äquidistant über die Länge der Heizmatte in Querrichtung parallel zueinander angeordnet. Werden bei dieser Ausgestaltung der Heizmatte 1 die Stromversorgungsleitungen 31 und 32, sowie 35 und 36 mit Strom beaufschlagt, so werden lediglich der linke und rechte Bereich der Heizmatte 1 erwärmt, wohingegen deren Mitte auf Raumtemperatur verbleibt.

Wahlweise können z.B. auch die Stromversorgungsleitungen 31 und 33 mit Strom beaufschlagt werden. Bei dieser Verwendung der Heizmatte würde der gesamte Bereich zwischen diesen beiden Stromversorgungsleitungen erwärmt.

In Figur 2 ist ein Teilschnitt durch ein erfindungsgemäß verwendetes Heizelement gezeigt. Die Stromversorgungsleitungen 3 sind als Lahnbänder ausgebildet. Diese erstrecken sich durch die Widerstandsmasse 4. Die Widerstandsmasse umfaßt bei der dargestellten Ausführungsform ein Gitter aus einem elektrisch nicht leitenden Material 10, dessen Fäden mit einer Schicht 12 aus einem elektrisch leitenden Polymer bedeckt sind. In der dargestellten Ausführungsform sind die einzelnen Gitterfäden mit dem Polymer beschichtet, wodurch die durch das Gitter gebildeten Öffnungen 13 verbleiben. Dadurch wird eine hohe Flexibilität des Widerstandsheizelementes erzielt und eine Dampfdiffusion durch die Heizmatte nicht behindert.

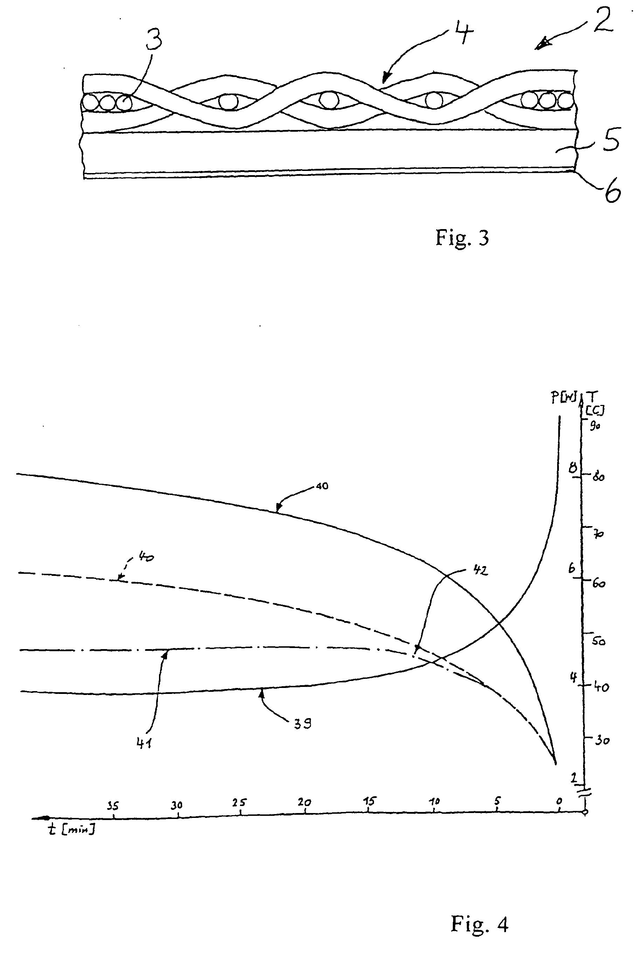

In Figur 3 ist ein Schnitt durch eine weitere Ausführungsform der erfindungsgemäßen Heizmatte dargestellt. Die Stromversorgungsleitungen 3 erstrecken sich durch die Widerstandsmasse 4, die als Gitter ausgebildet ist. An der Unterseite der Widerstandsmasse ist eine Isolierschicht 5 angeordnet. Auf der dem Widerstandsheizelement abgewandten Seite der Isolierschicht ist weiterhin eine Reflexionsschicht 6 vorgesehen. Die vom Widerstandsheizelement abgegebenen Strahlungen, die durch die Isolationsschicht hindurchdringen, werden an der Reflexionsschicht 6 reflektiert und in Richtung des Widerstandsheizelementes zurückgestrahlt.

In Figur 4 ist der Verlauf der Energieaufnahme in Abhängigkeit von der Zeit sowie der Verlauf der Temperatur im Vergleich zur Leistungsaufnahme eines erfindungsgemäßen Heizelementes dargestellt.

Wie die Kennlinie 39 zeigt, sinkt die Leistungsaufnahme des Widerstandsheizelementes 1 mit zunehmender Zeitdauer durch das Ansteigen der Temperatur und der damit erfolgenden Erhöhung des Widerstandes in der Widerstandsmasse 4. Dabei ergibt sich eine Selbststabilisierung des Widerstandsheizelementes bei einer durch das elektrisch leitende Polymer einstellbaren Grenztemperatur. Der Temperaturverlauf am Widerstandsheizelement bei idealer Wärmedämmung ist aus der durchgezogenen Kennlinie 40 zu ersehen. Die unterbrochene Kennlinie 40 zeigt den Temperaturverlauf des Widerstandsheizelementes bei Wärmeabgabe, z.B. bei Verwendung in der erfindungsgemäßen Heizmatte. Die Temperaturstabilisierung wird bei ca. 50°C erreicht. Wie in der Kennlinie 41 gezeigt, kann die Widerstandsmasse durch das elektrisch leitende Polymer auch derart eingestellt werden, daß sie einen nichtlinearen Temperaturkoeffizienten des elektrischen Widerstandes aufweist, wobei der Knick 42 in der Kennlinie 41 das sprunghafte Ansteigen des Widerstandes nach Erreichen dieser Grenztemperatur anzeigt. Dies bewirkt ein sprunghaftes Ansteigen des Widerstandes in der Widerstandsmasse und ein Absinken der Leistungsaufnahme, sodaß nach relativ kurzer Aufheizzeit eine rasche Temperaturstabilisierung im Widerstandsheizelement erfolgt. Auch diese Kennlinie stellt den Temperaturverlauf bei Wärmeabgabe insbesondere bei Verwendung als Heizmatte dar. Die Widerstandsmasse wird vorzugsweise so gewählt, daß der Kennlinienknick im Bereich der Körpertemperatur liegt. Bei einer solchen Widerstandsmasse treten keine hohen Temperaturen auf und die Heizmatte kann dennoch einen darauf oder darunter liegenden Körper insbesondere durch die Tiefenwirkung hinreichend erwärmen.

Im Rahmen der Erfindung liegt es ebenfalls, Stromversorgungsleitungen vorzusehen, die sich in Längsrichtung der Heizmatte erstrecken. Bei dieser Ausführungsform können Längsbereiche der Heizmatte durch Anlegen von Strom an die jeweiligen Stromversorgungsleitungen erwärmt werden und andere Längsbereiche bei Raumtemperatur verbleiben.

Besteht das Gitter der Widerstandsmasse aus Fäden, die vollständig aus einem elektrisch leitenden Polymer hergestellt sind, so kann ein gleichmäßiger Stromdurchgang durch die Fläche der Widerstandsmasse durch geeignete Wahl des Durchmessers der Fäden erzielt werden. Hierbei wird der Durchmesser der Fäden des Gitters, die parallel zu den Elektroden verlaufen, kleiner als der Durchmesser der senkrecht zu den Elektroden verlaufenden Fäden gewählt. Ein gleichmäßiger Stromdurchgang kann auch durch geeignete Wahl des Materials der Fäden erzielt werden. Hierbei wird das Material für die Fäden, die senkrecht zu den Elektroden verlaufen, so gewählt, daß dieses einen höheren Leitwert aufweist als das der parallel zu diesen verlaufenden Fäden. Hierbei kann eine Differenz in der Leitfähigkeit zwischen den parallel und den senkrecht zu den Elektroden verlaufenden Fäden von 15 - 25% vorzugsweise 20% ausreichen, um den Stromfluß durch die Fäden zu regeln und ideal über die gesamte Fläche zu verteilen, wodurch diese gleichmäßig erwärmt wird.

Es liegt auch im Rahmen der Erfindung, die Widerstandsmasse durch eine flexible Schicht zu bilden, die ein Stützmaterial umfaßt, daß mit dem elektrisch leitfähigen Polymer beschichtet oder getränkt ist, wodurch eine kontinuierliche Schicht gebildet wird. Das Stützmaterial kann ein Vlies, ein dichtes Gewebe oder eine Fasermatte sein. Dieses Stützmaterial wird vorzugsweise aus Polyamid, z.B. Nylon oder Polyester, oder Polypropylen hergestellt. Durch dieses Stützmaterial wird der Heizmatte zum einen eine gewisse Steifigkeit verliehen, die ein Knicken der Heizmatte verhindert. Zum anderen wird eine Fläche aus elektrisch leitendem Polymer gebildet. Diese Fläche besitzt eine relativ ebene Oberfläche, die lediglich durch die Materialstruktur geringe Vertiefungen aufweist. Stellen, an denen an der Oberfläche kein elektrisch leitendes Polymer vorliegt, weißt die Schicht nicht auf. Durch diese kontinuierliche Schicht wird von dem Heizelement eine einheitliche Wellenlänge abgegeben, die zu einer gleichmäßigen Erwärmung des Körpers führt. Dennoch kann das Stützgewebe durch Poren atmungsaktiv sein und Flüssigkeit hindurch lassen. Bei einem porösen Material sind die Poren lediglich an den Innenwänden mit elektrisch leitendem Polymer beschichtet, aber nicht vollständig gefüllt.

Eine solche kontinuierliche Widerstandsschicht kann durch Eintauchen des Stützgewebes in das elektrisch leitende Polymer oder durch Sprühtechniken hergestellt werden. Beim letzteren Verfahren kann auch nur die der Isolierschicht abgewandte Seite mit dem elektrisch leitenden Polymer beschichtet werden, wodurch das Stützmaterial als zusätzliche Isolierung dient.

Durch die bei der erfindungsgemäß eingesetzten Heizmatte benötigten geringen Spannungen ist es nicht notwendig, eine Feuchtigkeit abweisende Hülle oder Schicht um die Heizmatte vorzusehen. Auch eine Alterung ist bei der vorliegenden Heizmatte nicht zu befürchten, da anders als bei Rußleitern im erfindungsgemäß eingesetzten elektrisch leitfähigen Polymer keine Oxidation auftritt.

Zur Verbesserung des elektrischen Kontaktes zwischen der Widerstandsmasse und den Stromversorgungsleitungen kann die Widerstandsmasse im Bereich der Stromversorgungsleitungen mit einer aufgespritzten Schicht aus Metall metallisiert sein.

Die erfindungsgemäße Heizmatte kann in eine herkömliche Bettmatraze eingearbeitet sein. Die Heizmatte befindet sich dabei möglichst nahe an der Oberfläche der Matraze und ist vorzugsweise lediglich durch eine textile Schicht abgedeckt. Dadurch kann die Wärme ideal an den menschlichen Körper abgegeben werden. Die Stromversorgungsleitungen werden bei dieser Ausführungsform durch die Matraze geführt.

Die Heizmatte kann geringe Dicken von 0,5 - 2 cm aufweisen, wobei die Widerstandsschicht Dicken von 0,1 - 5 mm aufweist.

Die Heizmatte kann aufgrund ihrer Eigenschaften, wie der Möglichkeit der zonenweise Erwärmung, insbesondere in Krankenhäusern auf der Intensivstation, als Aufwachbett oder in der Geriatrie Anwendung finden. Insbesondere bei diesen Anwendungen kommt die durch die Heizmatte aufgrund des elektrisch leitenden Polymers hervorgerufene Abschirmwirkung gegen Strahlungen besonders zum Tragen.

Das erfindungsgemäß verwendete elektrisch leitende Polymer ist vorzugsweise durch Dotierung eines Polymers erzeugt. Die Dotierung kann eine Metall- oder Halbmetall-Dotierung sein. Bei diesen Polymeren ist der Störleiter chemisch an die Polymerkette gebunden und erzeugt eine Störstelle. Die Dotierungsatome und das Matrixmolekül bilden einen sogenannten Charge-Transfer Komplex. Bei der Dotierung werden Elektronen werden aus gefüllten Bändern des Polymers auf das Dotierungsmaterial übertragen. Durch die so entstandenen Elektronenlöcher erhält das Polymer halbleiterähnliche elektrische Eigenschaften. Durch chemische Reaktion wird bei dieser Ausführungsform ein Metall- oder Halbmetallatom so in die Polymerstruktur einbezogen bzw. an diese angelagert, daß hierdurch freie Ladungen erzeugt werden, die den Stromfluß entlang der Polymerstruktur ermöglichen. Die freien Ladungen liegen in Form von freien Elektronen oder Löchern vor. Es entsteht somit ein Elektronenleiter.

Vorzugsweise wurde das Polymer zum Dotieren mit einem Dotierungsmaterial in einer solchen Menge versetzt, daß das Verhältnis von Atomen des Dotierungsmaterials zu der Anzahl der Polymermoleküle mindestens 1:1, vorzugsweise zwischen 2:1 und 10:1, beträgt. Durch dieses Verhältnis wird erzielt, daß im wesentlichen alle Polymermoleküle zumindest mit einem Atom des Dotierungsmaterials dotiert sind. Durch Wahl des Verhältnisses kann der Leitwert der Polymere und dadurch der Widerstandsschicht, sowie der Temperaturkoeffizient des Widerstandes der Widerstandsschicht eingestellt werden.

Obwohl das erfindungsgemäß verwendete elektrisch leitende Polymer auch ohne Zusatz von Graphit in der erfindungsgemäßen Heizmatte als Material für die Widerstandsschicht eingesetzt werden kann, kann gemäß einer weiteren Ausführungsform die Widerstandschicht zusätzlich Graphitpartikel aufweisen. Diese Partikel können zu der Leitfähigkeit der gesamten Widerstandsschicht beitragen und berühren sich vorzugsweise nicht und bilden insbesondere keine Gitter- oder Skelettstrukturen aus. Die Graphitpartikel sind nicht fest in die Polymerstruktur eingebunden, sondern liegen frei beweglich vor. Befindet sich ein Graphitpartikel im Kontakt mit zwei Polymermolekülen, so kann der Strom von der einen Kette über das Graphit auf die nächste Kette überspringen.Die Leitfähigkeit der Widerstandsschicht kann so noch erhöht werden. Zugleich können die Graphitpartikel aufgrund ihrer freien Beweglichkeit in der Widerstandsschicht an die Elektroden gelangen und dort einen Verbesserung des Kontaktes bewirken.

Die Graphitpartikel liegen vorzugsweise in einer Menge von maximal 20 vol-%, besonders bevorzugt maximal 5 vol%, bezogen auf das Gesamtvolumen der Widerstandsschicht vor und weisen einen mittleren Durchmesser von maximal 0,1 µm auf. Durch diese geringe Menge an Graphit und den geringen Durchmesser kann das Ausbilden eines Graphitgitters, das zu einer Leitung des Stromes über diese Gitter führen würde vermieden werden. Es wird somit sicher gestellt, daß der Stromfluß weiterhin im wesentlichen über die Polymermoleküle durch Elektronen-Leitungen erfolgt und so die oben genannten Vorteile erzielt werden können. Insbesondere muß die Leitung nicht über ein Graphitgitter bzw. Skelett erfolgen, bei dem sich die Graphitpartikel berühren müssen und das bei mechanischer und thermischer Belastung leicht zerstört wird, sondern sie erfolgt entlang dem dehnbaren und alterungsbeständigen Polymer.

Als elektrisch leitende Polymere können sowohl elektrisch leitende Polymerisate wie Polystyrol, Polyvinylharze, Polyacrylsäure-Derivate und Mischpolymerisate derselben, als auch elektrisch leitende Polyamide und deren Derivate, Polyfluorkohlenwasserstoffe, Epoxyharze und Polyurethane verwendet werden. Bevorzugt können Polyamide, Polymethylmethacrylate, Epoxide, Polyurethane sowie Polystyrol oder Mischungen davon verwendet. Hierbei weisen Polyamide zusätzlich gute Klebeigenschaften auf, die für die Herstellung der erfindungsgemäßen Heizmatte von Vorteil sind, da hierdurch das Anbringen der Elektroden an der Widerstandsmasse bzw. der Isolierschicht erleichtert wird. Einige Polymere, wie z.B. Polyacetylene scheiden aufgrund ihrer geringen Alterungsbeständigkeit durch Reaktionsfreudigkeit mit Sauerstoff für den erfindungsgemäßen Einsatz aus.

Die Länge der verwendeten Polymermoleküle variiert in großen Bereichen abhängig von der Art und der Struktur des Polymers liegt aber vorzugsweise mindestens bei 500, besonders bevorzugt bei mindestens 4000 Å.

Als elektrisch leitendes Polymer können erfindungsgemäß in der Widerstandsfläche der Heizmatte insbesondere solche Polymere verwendet werden, die durch Metall- oder Halbmetallatome, die an die Polymere angelagert sind, leitfähig sind. Diese Polymere besitzen vorzugsweise einen spezifischen Durchgangswiderstand im Bereich der Werte, die von Halbleitern erzielt werden. Er kann bis zu 10² Ω·cm betragen, vorzugsweise liegt er höher, höchstens aber bei 10⁵ Ω·cm. Solche Polymere können durch ein Verfahren erhalten werden, bei dem Polymer-Dispersionen, Polymer-Lösungen oder Polymere mit Metall- oder Halbmetallverbindungen oder deren Lösung in einer Menge versetzt werden, so daß auf ein Polymer-Molekül annähernd ein Metall- oder Halbmetallatom kommt. Dieser Mischung wird ein Reduktionsmittel in geringem Überschuß zugegeben oder durch bekannte thermische Zersetzung Metall- oder Halbmetallatome gebildet. Anschließend werden die gebildeten oder noch vorhandenen Ionen ausgewaschen und die Dispersionslösung oder das Granulat kann gegebenenfalls mit Graphit oder Ruß versetzt werden.

Die erfindungsgemäß eingesetzten elektrisch leitenden Polymere sind vorzugsweise frei von Ionen. Maximal beträgt der Gehalt an freien Ionen l Gew.% bezogen auf das Gesamtgewicht der Widerstandsschicht. Die Ionen werden entweder wie oben beschrieben ausgewaschen oder es wird ein geeignetes Reduktionsmittel zugegeben. Das Reduktionsmittel wird in einem solchen Verhältnis zugegeben, daß die Ionen vollständig reduziert werden können. Der geringe Anteil an Ionen, vorzugsweise die Ionenfreiheit der erfindungsgemäß verwendeten elektrisch leitenden Polymere bewirkt eine lange Beständigkeit der Widerstandsschicht unter Einwirkung von elektrischen Strömen. Wie sich gezeigt hat, besitzen Polymere, die Ionen zu einem höheren Prozentsatz enthalten, eine nur geringe Alterungsbeständigkeit bei Einwirkung von elektrischen Strömen, da es durch Elektroylse-Reaktionen zur Selbstzerstörung der Widerstandsschicht kommt. Das erfindungsgemäß verwendete elektrisch leitende Polymer hingegen ist aufgrund der geringen Ionenkonzentration auch bei längerer Beaufschlagung mit Strom alterungsbeständig. Als Reduktionsmittel für das oben beschriebene Verfahren zur Herstellung eines erfindungsgemäß eingesetzten elektrisch leitenden Polymers werden solche Reduktionsmittel verwendet, die entweder keine Ionen bilden, weil sie thermisch bei der Verarbeitung zersetzt werden, wie z.B. Hydrazin, oder mit dem Polymer selbst chemisch reagieren, wie z.B. Formaldehyd oder solche, deren Überschuß oder Reaktionsprodukte sich leicht auswaschen lassen. wie z.B. Hypophosphite. Als Metall oder Halbmetalle werden vorzugsweise Silber, Arsen, Nickel, Graphit oder Molybdän verwendet. Besonders bevorzugt sind solche Metall oder Halbmetallverbindungen, die durch reine thermische Zersetzung das Metall oder Halbmetall ohne störende Reaktionsprodukte bilden. Insbesondere Arsenwasserstoff oder Nickelcarbonyl haben sich als besonders vorteilhaft erwiesen. Die erfindungsgemäß verwendeten elektrisch leitenden Polymere können z.B. hergestellt werden, indem das Polymer mit 1-10 Gew-% (bezogen auf das Polymer) einer Vormischung, die nach einer der folgenden Rezepturen hergestellt wurde, versetzt wird.
- Beispiel 1:: 1470 Gew.Teile Dispersion von Fluorkohlenwasserpolymers (55 % Feststoff in Wasser), 1 Gew.-Teil Netzmittel, 28 Gew.-Teile Silbernitratlösung 10 %, 6 Gew.-Teile Kreide, 8 Gew.-Teile Ammoniak, 20 Gew.-Teile Ruß, 214 Gew.-Teile Graphit, 11 Gew.-Teile Hydrazinhydrat.
- Beispiel 2:: 1380 Gew.-Teile Acrylharzdispersion 60 Gew.-% in Wasser, 1 Gew.-Teil Netzmittel, 32 Gew.-Teile Silbernitratlösung 10 %ig, 10 Gew.-Teile Kreide, 12 Gew.-Teile Ammoniak, 6 Gew.-Teile Ruß, 310 Gew.-Teile Graphit, 14 Gew.-Teile Hydrazinhydrat.
- Beispiel 3:: 2200 Gew.-Teile dest. Wasser, 1000 Gew.-Teile Styrol (monomer), 600 Gew.-Teile Ampholytseife (15 %ig), 2 Gew.-Teile Natriumpyrophosphat, 2 Gew.-Teile Kaliumpersulfat, 60 Gew.-Teile Nickelsuflat, 60 Gew.-Teile Natriumhypophospit, 30 Gew.-Teile Adipinsäure, 240 Gew.-Teile Graphit.

## Patentansprüche

1. Heizmatte (1) mit einem flächigen Widerstandsheizelement (2), dessen Widerstandsmasse (4) ein elektrisch leitendes Polymer umfasst, wobei in dem Widerstandsheizelement (2) drei oder mehr Stromversorgungsleitungen (3) vorgesehen sind, die wahlweise mit Strom beaufschlagt werden können, und die Widerstandsmasse (4) ein Gitter umfaßt, wobei die Fäden des Gitters aus einem Kunststoff aus dem elektrisch leitenden Polymer gebildet sind oder die Fäden aus einem anderen Material bestehen und mit diesem Kunststoff beschichtet sind.

2. Heizmatte gemäß Anspruch 1, **dadurch gekennzeichnet, daß** das elektrisch leitende Polymer einen positiven Temperaturkoeffizienten des elektrischen Widerstandes aufweist.

3. Heizmatte gemäß Anspruch 1 oder 2, **dadurch gekennzeichnet, daß** sich die Widerstandsmasse des Widerstandsheizelementes in Längsrichtung in der Heizmatte (1) erstreckt und die Stromversorgungsleitungen (3) sich in Breitenrichtung durch die Widerstandsmasse (4) erstrecken, so daß der an den Stromversorgungsleitungen (3) angelegte Strom die Widerstandsmasse (4) senkrecht zu der Dicke der Widerstandsmasse (4) durchfließt.

4. Heizmatte gemäß einem der Ansprüche 2 bis 3, **dadurch gekennzeichnet, daß** die Widerstandsmasse einen nicht linearen positiven Temperaturkoeffizienten des elektrischen Widerstandes mit einem Kennlinienknick aufweist.

5. Heizmatte gemäß einem der vorstehenden Ansprüche, **dadurch gekennzeichnet, daß** die Stromversorgungsleitungen (3) durch Lahnbänder gebildet werden.

6. Heizmatte gemäß einem der vorstehenden Ansprüche, **dadurch gekennzeichnet, daß** die Stromversorgungsleitungen (3) parallel zueinander in einem Abstand von mindestens 4 cm angeordnet sind.

7. Heizmatte gemäß einem der vorstehenden Ansprüche, **dadurch gekennzeichnet, daß** Einrichtungen vorgesehen sind, um die Stromversorgungsleitungen (3) wahlweise an eine einzige Stromquelle anschließen zu können.

8. Heizmatte gemäß einem der vorstehenden Ansprüche, **dadurch gekennzeichnet, daß** nur an einer Seite des Widerstandsheizelementes (2) eine Isolierschicht (5) angeordnet ist.

9. Heizmatte gemäß einem der vorstehenden Ansprüche, **dadurch gekennzeichnet, daß** an einer Seite des Widerstandsheizelementes (2) eine Reflexionsschicht (6) angeordnet ist.

## Revendications

1. Couverture chauffante (1) avec une résistance chauffante plate (2) dont la masse de résistance (4) comprend un polymère électroconducteur, trois ou plusieurs lignes d'alimentation électrique (3), qui peuvent être alimentées en courant, au choix, étant prévues dans la résistance chauffante (2) et la masse de résistance (4) comprenant une grille, les fils de la grille étant constitués d'une matière synthétique à partir du polymère électroconducteur ou les fils étant constitués d'une autre matière et étant enduits avec cette matière synthétique.

2. Couverture chauffante selon la revendication 1, **caractérisée en ce que** le polymère électroconducteur présente un coefficient de température positif de la résistance électrique.

3. Couverture chauffante selon la revendication 1 ou 2, **caractérisée en ce que** la masse de résistance de la résistance chauffante s'étend dans le sens longitudinal dans la couverture chauffante (1) et les lignes d'alimentation électrique (3) s'étendent dans le sens de la largeur à travers la masse de résistance (4), de sorte que le courant appliqué aux lignes d'alimentation électrique (3) parcoure la masse de résistance (4) perpendiculairement à l'épaisseur de la masse de résistance (4).

4. Couverture chauffante selon l'une quelconque des revendications 2 à 3, **caractérisée en ce que** la masse de résistance (4) présente un coefficient de température positif non linéaire de la résistance électrique avec un coude de courbe caractéristique.

5. Couverture chauffante selon l'une quelconque des revendications précédentes, **caractérisée en ce que** les lignes d'alimentation électrique (3) sont formées par des lamelles de métal.

6. Couverture chauffante selon l'une quelconque des revendications précédentes, **caractérisée en ce que** les lignes d'alimentation électrique (3) sont disposées parallèlement les unes aux autres à une distance d'au moins 4 cm.

7. Couverture chauffante selon l'une quelconque des revendications précédentes, **caractérisée en ce qu'**il est prévu des dispositifs permettant de raccorder les lignes d'alimentation électrique (3), au choix, à une seule source de courant.

8. Couverture chauffante selon l'une quelconque des revendications précédentes, **caractérisée en ce qu'**une couche isolante (5) est disposée d'un seul côté de la résistance chauffante (2).

9. Couverture chauffante selon l'une quelconque des revendications précédentes, **caractérisée en ce qu'**une couche réflecteur (6) est disposée d'un côté de la résistance chauffante (2).

## Claims

1. Heating blanket (1) with a flat resistance heating element (2) whose resistance mass (4) comprises an electrically conductive polymer, three or more current-supply lines (3) being provided in the resistance heating element (2), which can optionally be charged with current, and the resistance mass (4) comprising a lattice, the filaments of the lattice being formed from a plastic from the electrically conductive polymer or the filaments consisting of another material and being coated with this plastic.

2. Heating blanket according to claim 1, **characterized in that** the electrically conductive polymer displays a positive temperature coefficient of the electric resistance.

3. Heating blanket according to claim 1 or 2, **characterized in that** the resistance mass of the resistance heating element extends in longitudinal direction in the heating blanket (1) and the current-supply lines (3) extend in width direction through the resistance mass (4), with the result that the current applied to the current-supply lines (3) flows through the resistance mass (4) perpendicular to the thickness of the resistance mass (4).

4. Heating blanket according to one of claims 2 to 3, **characterized in that** the resistance mass displays a non-linear positive temperature coefficient of the electric resistance with a kink in the characteristic curve.

5. Heating blanket according to one of the previous claims, **characterized in that** the current-supply lines (3) are formed by tinsel strips.

6. Heating blanket according to one of the previous claims, **characterized in that** the current-supply lines (3) are arranged parallel to one another spaced apart by at least 4 cm.

7. Heating blanket according to one of the previous claims, **characterized in that** equipment is provided in order to be able to connect the current-supply lines (3) optionally to a single power source.

8. Heating blanket according to one of the previous claims, **characterized in that** an insulating layer (5) is arranged on only one side of the resistance heating element (2).

9. Heating blanket according to one of the previous claims, **characterized in that** a reflecting layer (6) is arranged on one side of the resistance heating element (2).
